(19) Europäisches Patentamt

European Patent Office

Office européen des brevets

(11) **EP 4 353 275 B1**

(12) **EUROPEAN PATENT SPECIFICATION**

(45) Date of publication and mention
of the grant of the patent:
**03.06.2026  Bulletin 2026/23**

(21) Application number: **22201283.3**

(22) Date of filing: **13.10.2022**

(51) International Patent Classification (IPC):
***A61M 1/16*** *(2006.01)*     ***A61M 1/34*** *(2006.01)*
***A61M 1/36*** *(2006.01)*

(52) Cooperative Patent Classification (CPC):
**A61M 1/16; A61M 1/34; A61M 1/3413;
A61M 1/3496; A61M 1/3609; A61M 1/3656;
A61M 1/3666;** A61M 2205/3331; A61M 2205/6018

(54) **APPARATUS FOR EXTRACORPOREAL BLOOD TREATMENT**

VORRICHTUNG ZUR EXTRAKORPORALEN BLUTBEHANDLUNG

APPAREIL DE TRAITEMENT EXTRACORPOREL DU SANG

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(43) Date of publication of application:
**17.04.2024  Bulletin 2024/16**

(73) Proprietor: **Gambro Lundia AB
226 43 Lund (SE)**

(72) Inventor: **POUCHOULIN, Dominique
F-01390 Tramoyes (FR)**

(74) Representative: **PGA S.p.A., Milano, Succursale
di Lugano
Via Castagnola, 21c
6900 Lugano (CH)**

(56) References cited:
**EP-B1- 2 941 282      US-A1- 2004 084 358
US-A1- 2019 111 199**

## Description

### Technical Field

The present invention relates to an apparatus for extracorporeal blood treatment capable of detecting if the respective extracorporeal blood circuit is connected to a vascular access of a patient or to a blood circuit of a high blood flow rate machine. The present invention further relates to a combination of said apparatus for extracorporeal blood treatment with a high blood flow rate machine. The present invention is applicable in the context of medical apparatuses and methods for extracorporeal blood treatment, such as for continuous renal replacement therapies (CRRT) in Intensive Care Units (ICU), for hemoperfusion or for Therapeutic Plasma Exchange (TPE).

### Background of the Invention

CRRT systems are configured for delivering treatments designed for patients versing in acute states of illness and who may have temporarily lost their kidney function in its entirety. CRRT monitors should be able to deliver various therapies, e.g.: ultrafiltration (UF), continuous veno venous hemofiltration (CCVH), continuous veno venous hemodiffiltration (CWHDF), continuous veno venous hemodialysis (CCVHD). During the therapy, blood flows in/out from the body and the average blood flow rate is usually between 100 ml/min and 250 ml/min in adults and below 100 ml/min in pediatric applications.

Hemoperfusion is a process whereby blood is passed through a sorbent system to remove specific toxic substances in the blood. The sorbent system may be made up of a plastic housing, or cartridge, which contains granules that allow adsorption of molecules. During this therapy, blood flows in/out from the body and the average blood flow rate is usually between 50 ml/min and 200 ml/min.

Therapeutic plasma exchange (TPE), also termed as plasmapheresis, is a process involving extracorporeal removal of plasma from other components of blood by a cell separator, discarding and replacing plasma with physiological fluids. During this therapy, blood flows in/out from the body and the average blood flow rate is usually between 100 ml/min and 250 ml/min.

CRRT, TPE and Hemoperfusion systems may be combined with machines with high blood flow circuits, like Extra Corporeal Membrane Oxygenation (ECMO) or heart-lung machines for cardiopulmonary bypass, which are used to support the heart and/or lung function in patients with severe cardiac and/or respiratory failure.

Indeed, acute kidney injury is common in patients receiving ECMO. The most common reasons for starting dialysis on ECMO are fluid overload (43%), prevention of fluid overload (16%), acute kidney injury (35%) and electrolyte disturbances (4%). The early introduction of CRRT may prevent fluid overload and is often used in clinical practice. A blood flow rate of ECMO machines and other high blood flow rate machines, like Extracorporeal Life Support (ECLS) machines or heart-lung machines for cardio-pulmonary by-pass, may be 2 L/min - 5 L/min, which is much higher than blood flow of CRRT systems.

Several prior art documents disclose the combination of ECMO machines and apparatuses for extracorporeal blood treatment.

For instance, document US2010288703A1 discloses an ECMO - CVVH (continuous veno-venous hemofiltration) system, wherein the CWH device may be utilized as a stand-alone CVVH device or within the CWH portion of the combined ECMO-CWH system.

Document CN113230531A discloses a combination of ECMO and CRRT wherein the input end of the CRRT is connected downstream the oxygenator and the output end of the CRRT is connected upstream the oxygenator. A pressure regulating device comprising pressure sensors and flow rate sensors is used to detect and to control flow rates and pressures between the ECMO and the CRRT. The cited combination is also disclosed by documents US2022080093A1 and CN212817420U.

The apparatuses for extracorporeal blood treatment may be connected in multiple ways to an established ECMO circuit, leading to the possibility of running the extracorporeal blood treatment therapy with conditions of positive access pressure and/or negative return pressure which are unusual with respect to the conditions typical of the direct connection of the apparatuses for extracorporeal blood treatment to the vascular access of a patient through a catheter.

These unusual conditions may cause the control procedures of the apparatuses for extracorporeal blood treatment based on pressure monitoring methods to work improperly or to fail. For instance, the detection of access or return disconnection events through pressure monitoring methods may fail if the apparatus for extracorporeal blood treatment is connected to an ECMO machine, since pressure changes at disconnection may become minimal according to the operating ECMO pressures and further to the lack of pressure drop at the connecting point (no catheter pressure drop).

Documents US20040084358A1, US20190111199A1 and EP2941282B1 disclose extracorporeal blood treatment apparatuses.

## Summary

In this situation, it is an object of the present invention to offer a technical solution capable overcoming one or more of the above drawbacks.

It is an object of the present invention to secure compatibility of an apparatus for extracorporeal blood treatment, like CRRT, TPE, hemoperfusion, with a high blood flow rate machine, like an Extra Corporeal Membrane Oxygenation (ECMO) machine or an Extracorporeal Life Support (ECLS) machine or a heart-lung machine for cardio-pulmonary by-pass, such that the apparatus and the machine are able to run simultaneously the respective therapies.

It is also an aim of the present invention to ensure that the apparatus for extracorporeal blood treatment works properly both when connected to a vascular access of a patient or to a blood circuit of a high blood flow rate machine.

It is also an aim of the present invention to improve the safety for the patient and the operator/s when the patient undergoes simultaneously dialysis and a high blood flow rate treatment, like ECMO.

It is also an aim of the present invention to ensure that the setup of the extracorporeal blood treatment is correct both when the apparatus is connected to the vascular access of the patient and when the apparatus is connected to the high blood flow rate machine.

It is also an aim of the present invention to adapt the working parameters of the apparatus and, particularly, the pressures operating ranges according to the type of connection.

It is also an aim of the present invention to control the pressure monitoring system according to the type of connection, e.g. in order to assure that disconnection events are detected correctly or to prevent triggering unnecessary alarms.

According to the safety aspects discussed above, it is also an aim of the present invention to secure that the blood access configuration made by the operator is correct.

At least one of the above objects is substantially reached by an apparatus for extracorporeal blood treatment according to one or more of the appended claims.

One or more of the above objects is also substantially reached by a combination of an apparatus for extracorporeal blood treatment and a high blood flow rate machine according to one or more of the appended claims.

The apparatus herein disclosed allows to automatically identify the type of connection of its extracorporeal blood circuit, i.e. to detect if the extracorporeal blood circuit is connected to a vascular access of a patient or to a blood circuit of a high blood flow rate machine.

## Description of the drawings

Aspects of the invention are shown in the attached drawings, which are provided by way of non-limiting examples, wherein:

Figure 1 shows a schematic representation of an apparatus for extracorporeal blood treatment connected to a patient;
Figure 2 shows a schematic view in side elevation of the apparatus for extracorporeal blood treatment of Figure 1;
Figure 3 shows a combination of the apparatus for extracorporeal blood treatment of Figures 1 and 2 with a high blood flow rate machine;
Figures 4 to 6 show different connections between the apparatus for extracorporeal blood treatment and the high blood flow rate machine;
Figure 7A shows a detailed and enlarged view of element of the apparatus for extracorporeal blood treatment of Figure 2;
Figure 7B shows an enlarged detail of the combination of Figure 3;

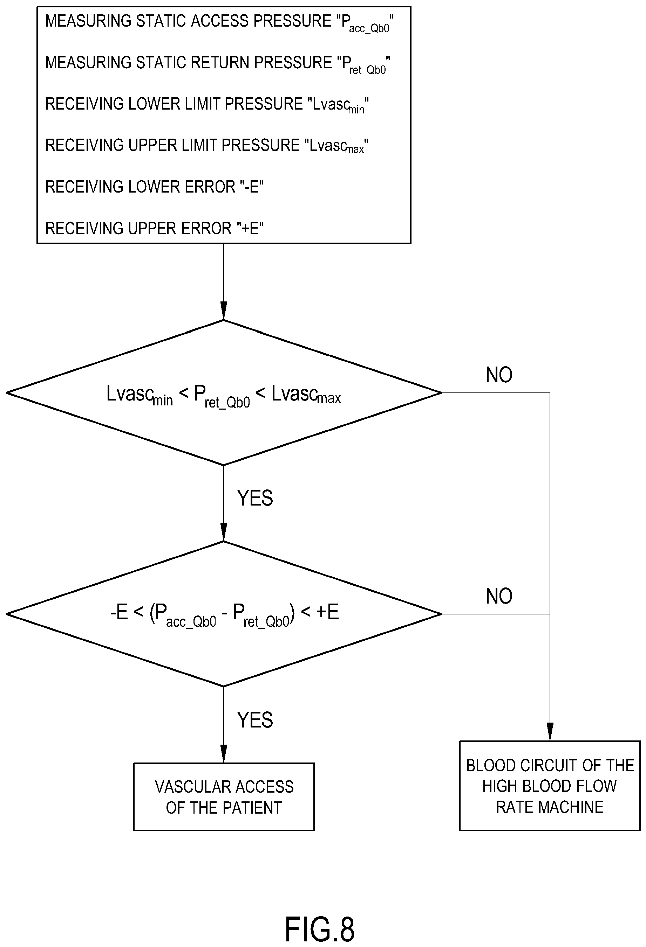

Figure 8 is a flowchart showing a method of detecting a type of connection of the apparatus for extracorporeal blood treatment;

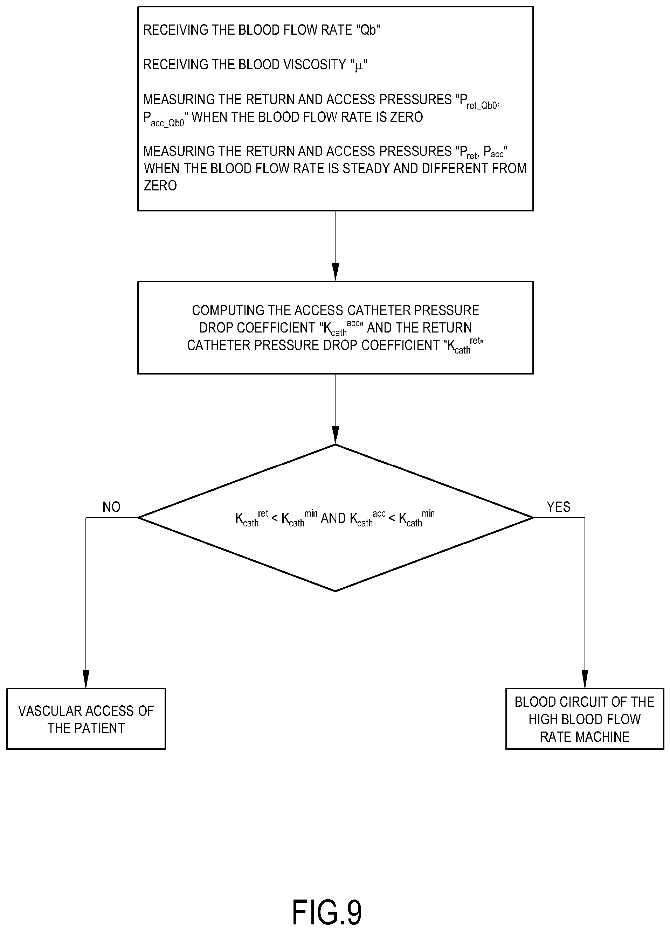

Figure 9 is a flowchart showing another method of detecting the type of connection of the apparatus;
Figure 10 schematically shows a high blood flow rate machine to be combined with the apparatus for extracorporeal blood treatment.

## Detailed description

### Apparatus for extracorporeal blood treatment

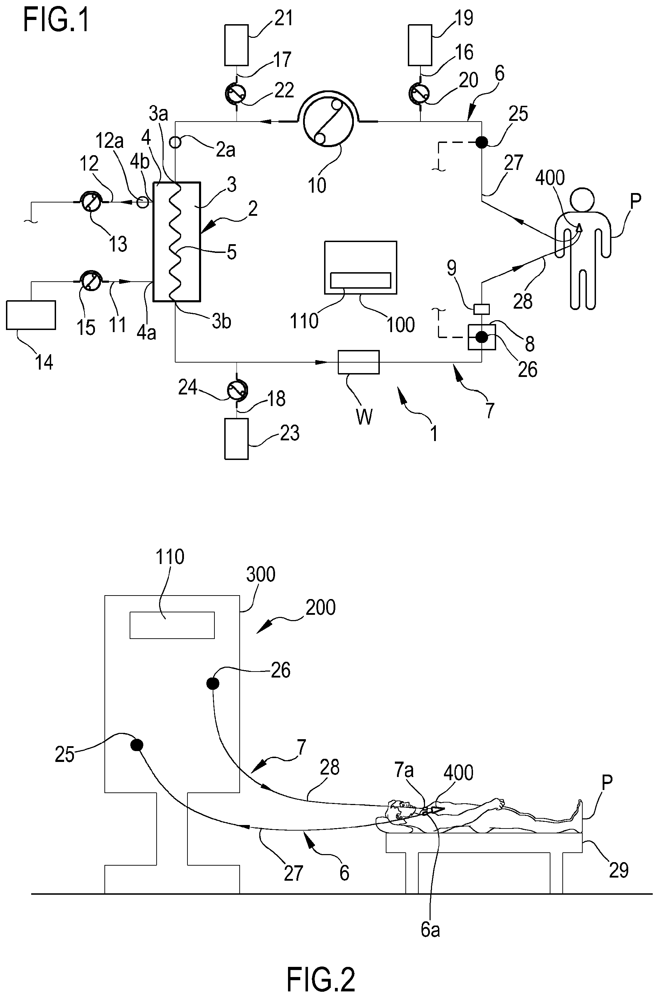

An apparatus 1 for extracorporeal blood treatment is schematically represented in Figure 1. The apparatus 1 is a continuous renal replacement therapy (CRRT) apparatus for intensive care treatments, for instance configured to deliver various therapies, like CCVH, CWHDF, CWHD, SCUF.

In other embodiments, not depicted in the attached drawings, the apparatus 1 for extracorporeal blood treatment may be a hemoperfusion machine (adsorber) or a machine for Therapeutic Plasma Exchange (TPE).

The apparatus 1 comprises a treatment unit 2, which in the embodiment of the attached drawings is a filtration unit having a primary chamber 3 and a secondary chamber 4 separated by a semi-permeable membrane 5. Depending upon the treatment, the semi-permeable membrane 5 of the treatment unit 2 may be selected to have different properties and performances.

A blood circuit is coupled to the primary chamber 3 of the treatment unit 2. The blood circuit comprises a blood withdrawal line 6 connected to an inlet 3a of the primary chamber 3, a blood return line 7 connected to an outlet 3b of the primary chamber 3. The blood withdrawal line 6 and blood return line 7 are configured for connection to a vascular access of a patient "P".

In use, the blood withdrawal line 6 and the blood return line 7 are connected to a vascular access of the patient "P". In detail, the blood withdrawal line 6 and the blood return line 7 are connected a vascular access device 400 which is then placed in fluid communication with the patient "P" vascular system, such that blood may be withdrawn through the blood withdrawal line 6, flown through the primary chamber 3 and then returned to the patient's vascular system through the blood return line 7. The vascular access device 400 may comprise two catheters placed in central vein(s) of the patient. Needles ends may be employed for other kind of apparatuses, like TPE.

An air detector, not shown, and an air separator, such as a deaeration chamber 8, may be present on the blood return line 7. Moreover, a monitor valve 9 may be present on the blood return line 7, downstream the deaeration chamber 8.

The blood flow through the blood circuit is controlled by a blood pump 10, for instance a peristaltic blood pump, acting either on the blood withdrawal line 6 or on the blood return line 7. The embodiment of Figure 1 shows the blood pump 10 coupled to a pump section of the blood withdrawal line 6.

A dialysis circuit is connected to the secondary chamber 4 of the treatment unit 2 and comprises a dialysis line 11 connected to an inlet 4a of the secondary chamber 4 and an effluent line 12 connected to an outlet 4b of the secondary chamber 4 and to a drain, not shown.

An effluent pump 13 is located on the effluent line 12 and is able to recall fluid from the second chamber 4. The dialysis line 11 is connected to a source 14, e.g. a bag or a preparation device, of fresh dialysis fluid and a dialysis pump 15 is located on the dialysis line 11 and is able to pump fluid to the second chamber 4.

The apparatus 1 further comprises an infusion circuit comprising at least one infusion line. The infusion circuit shown in the embodiment of Figure 1 comprises a pre-blood pump line 16, a pre-infusion line 17 and a post-infusion line 18. The pre-blood pump line 16 is connected to the blood withdrawal line 6 upstream of the blood pump 10 and to a first source 19 of infusion fluid, e.g. a bag. A pre-blood pump 20 is located on the pre-blood pump line 16 and is able to pump fluid from the first source 19 to the blood circuit.

The pre-infusion line 17 is connected to the blood withdrawal line 6 downstream of the blood pump 10 and upstream of the treatment unit 2 and to a second source 21 of infusion fluid, e.g. a bag. A pre-infusion pump 22 is located on the pre-infusion line 17 and is able to pump fluid from the second source 21 to the blood circuit.

The post-infusion line 18 is connected to the blood return line 7 downstream of the treatment unit 2 and to a third source 23 of infusion fluid, e.g. a bag. A post-infusion pump 24 is located on the post-infusion line 18 and is able to pump fluid from the third source 23 to the blood circuit.

The apparatus 1 may also comprise one or more auxiliary line/s, not shown, connected to the blood circuit and to a source of at least one compensation substance or of an anticoagulant, such as calcium or heparin, and a pump or syringe configured to deliver a flow rate of the compensation substance, such as calcium or heparin, etc.

An access pressure sensor 25 is configured to detect a pressure at a measurement location in the blood withdrawal line 6. A return pressure sensor 26 is configured to detect a pressure at a measurement location in the blood return line 7. The access pressure sensor 25 and the return pressure sensor 26 may comprise pressure pods in the blood withdrawal line 6 and blood return line 7. The return pressure sensor 26 may be operatively coupled to the deaeration chamber 8, as shown in Figure 1.

A filter pressure sensor 2a is positioned just upstream of the inlet 3a of the primary chamber 3 and is configured to measure pressure required to push blood through the treatment unit 2. An effluent pressure sensor 12a is positioned just downstream of the outlet 4b of the secondary chamber 4 and is configured to measure pressure required to push/pull ultrafiltrate from the blood.

The apparatus 1 may also comprise a blood warming device "W" coupled to the blood return line 7 and configured to warm blood before returning to the patient "P". The apparatus 1 may also comprise a fluid warmer, not shown in the Figures, coupled to one or more of the dialysis line 11, the pre-blood pump line 16, the pre-infusion line 17, the post-infusion line 18 and configured to warm the respective fluid.

A control unit 100 is connected and controls the blood pump 10, the dialysis pump 15, the effluent pump 13, the pre-blood pump 20, the pre-infusion pump 22 and the post-infusion pump 24 to regulate a blood flow rate "$Q_b$" in the blood circuit, a dialysis flow rate crossing the dialysis line 11, an effluent flow rate crossing the effluent line 12, an infusion flow rate crossing the pre-blood pump line 16, an infusion flow rate crossing the pre-infusion line 17, an infusion flow rate crossing the post-infusion line 18. Through the control of the dialysis flow rate crossing the dialysis line 11 and/or of the effluent flow rate crossing the effluent line 12, the control unit 100 is also configured to control/regulate a filtration flow rate (through the control of the dialysis pump 15 and the effluent pump 13) in the treatment unit 2 and/or a patient fluid removal rate (also through the control of the pre-blood pump 20, the pre-infusion pump 22 and the post-infusion pump 24).

The blood pump 10, controlled by the control unit 100, is configured to generate in the extracorporeal blood circuit an average blood flow rate "$Q_b$" between 100 ml/min and 250 ml/min during the therapy of adults and below 100 ml/min in pediatric applications.

The control unit 100 is also connected to the access pressure sensor 25, to the return pressure sensor 26 to the filter pressure sensor 2a and to the effluent pressure sensor 12a to receive signals correlated to pressure values from these sensors 2a, 12a, 25, 26. The access pressure sensor 25, the return pressure sensor 26 and the filter pressure sensor 2a provide to the control unit 100 signals correlated to pressure in the extracorporeal blood circuit.

The control unit 100 is also connected to and controls the blood warming device "W" and the fluid warmer(s).

The control unit 100 may be an electronic control unit comprising at least a CPU, a memory and input/output devices. The control unit 100 comprises or is connected to an interface 110 configured to display data and/or allow an operator to input data. For instance, the interface comprises a display, e.g. a touch screen, and/or buttons or a keyboard.

The apparatus 1 may comprise a treatment machine 200 and an integrated disposable set configured to be coupled to the treatment machine 200. The outline of the treatment machine 200 is represented schematically in Figure 2. The treatment machine 200 comprises the cited blood pump 10, effluent pump 13, dialysis pump 15, pre-blood pump 20, pre-infusion pump 22, post-infusion pump 24, control unit 100 with the interface 110, flow rate sensors.

The treatment machine 200 may comprise also the access pressure sensor 25 and the return pressure sensor 26 or the access pressure sensor 25 and the return pressure sensor 25 may be part of the integrated disposable set. The treatment machine 200 comprises also all the other elements and/or devices configured to receive and hold parts of the integrated disposable set.

The integrated disposable set comprises the treatment unit 2, the blood circuit, the effluent line 12, the dialysis line 11, the infusion lines 16, 17, 18, which are grouped together or which are to be connected together before use. When the integrated disposable set is mounted on the treatment machine 200, the access pressure sensor 25 and the return pressure sensor 26 or the measurement locations of the access pressure sensor 25 and return pressure sensor 26 are in fixed positions on a frame 300 of the treatment machine 200 and at predefined heights above the ground.

A section 27 of the blood withdrawal line 6 extends from the access pressure sensor 25 on the treatment machine 200 to the vascular access device 400 and to the patient "P" undergoing treatment. A section 28 of the blood return line 7 extends from the return pressure sensor 26 on the treatment machine 200 to the vascular access device 400 and to the patient "P".

As shown in Figure 2, the patient "P" undergoing treatment is lying on a bed 29 and the vascular access device 400 is placed at a height which may be different from a height of a location of measurement of the access pressure performed through the access pressure sensor 25 and of a location of measurement of the return pressure performed through the return pressure sensor 26 and may vary with respect to both the heights of these measurement locations, since the bed height is typically adjustable and/or the patient "P" may move or be moved.

**Vascular access device**

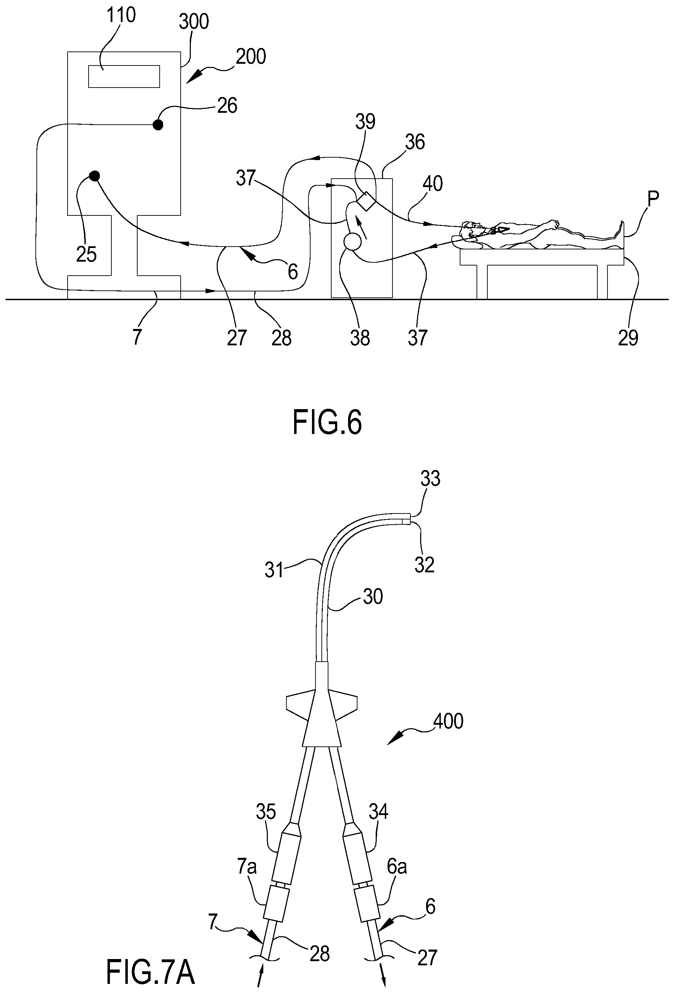

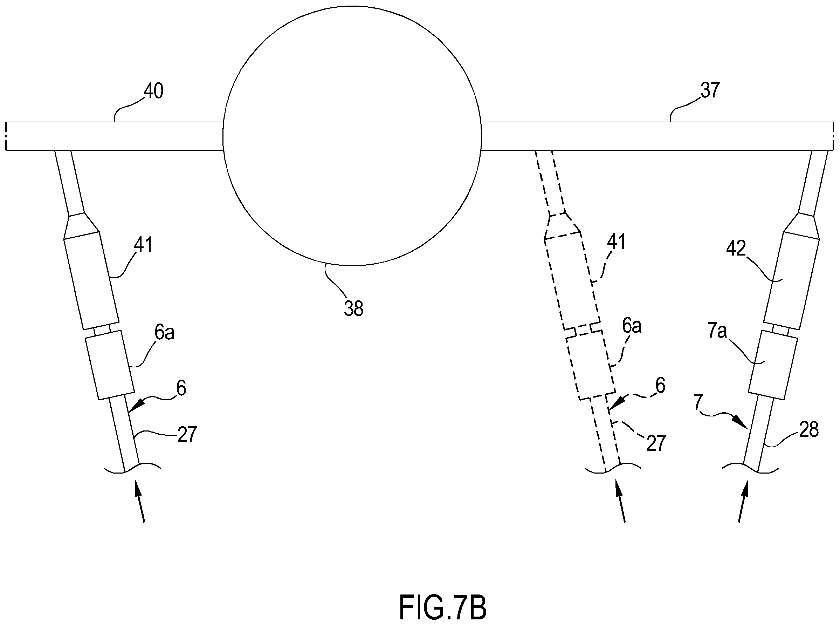

The vascular access device 400 shown in Figure 7 is central double lumen venous catheter (CVC) which is configured to be placed in a large central vein of the patient "P", for instance in the neck (internal jugular vein).

The vascular access device 400 is a double lumen catheter and comprises a withdrawal section 30 delimiting a withdrawal lumen and a return section 31 delimiting a return lumen. Distal portions of the withdrawal section 30 and return section 31 are provided respectively with a distal tip 32 of the withdrawal section 30 and a distal tip 33 of the return section. The distal portions of the withdrawal section 30 and return section 31 are paired and configured to be placed inside the large central vein. Proximal portions of the withdrawal section 30 and return section 31 are split and configured to remain outside the patient body.

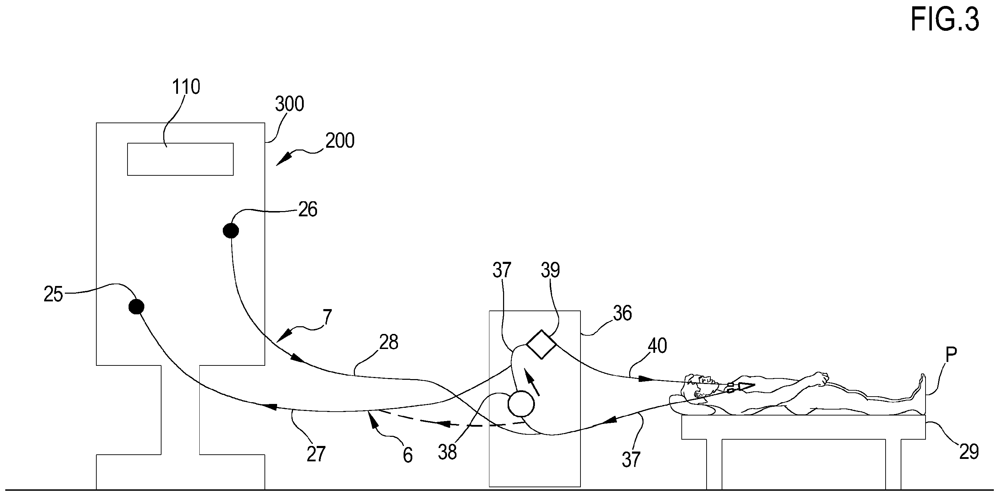

The proximal portions are provided respectively with a withdrawal port 34 and a return port 35. The withdrawal port 34 is connected or configured to be connected to a connector 6a of the blood withdrawal line 6 and the return port 35 is connected or configured to be connected to a connector 7a of the blood return line 7, as shown in Figure 3, and allow to connect the extracorporeal blood circuit to the vascular system of the patient "P". The connector and the respective port may be a Luer lock.

**Combination with Extra Corporeal Membrane Oxygenation (ECMO) machine**

In Figure 3, the apparatus 1 for extracorporeal blood treatment is coupled to a high blood flow rate machine 36. For instance, the high blood flow rate machine 36 is an Extra Corporeal Membrane Oxygenation (ECMO) machine. ECMO is an extracorporeal technique of providing prolonged respiratory (Veno-Venous (V-V) ECMO and Veno-Arterial (V-A) ECMO), and optionally cardiac (V-A ECMO), support to persons whose heart and lungs are unable to provide an adequate amount of gas exchange or perfusion to sustain life. The technology for ECMO is largely derived from cardiopulmonary bypass, which provides shorter-term support with arrested native circulation. Unlike a heart-lung bypass machine, which is designed for short-term use (during heart surgery, for instance), ECMO machines provide long-term heart and lung support over a period of hours, days, or even weeks to give a patient's heart and lungs time to heal and regain function.

The structure of the high blood flow rate machine 36 is per se known and is only schematically shown in Figure 3. The high blood flow rate machine 36 comprises: a venous line 37 with a venous access cannula that drains the venous blood, a pump 38, e.g. a small volume centrifugal pump, an oxygenator 39, an ultrasound sensor to measure the rate of blood flow as well as to act as a bubble detector (not shown in drawings), an oxygenated line 40 returning back to the patient and a return (arterial) cannula either back to the internal jugular vein or femoral vein in case of Veno-Venous ECMO (VV-ECMO) or the aorta in case of Veno-Arterial ECMO (VA-ECMO). The venous line 37 and the oxygenated line 40 are part of a blood circuit of the high blood flow rate machine 36. The venous line 37 extends from the patient "P" to the oxygenator 39. The oxygenated line 40 extends from the oxygenator 39 back to the patient "P".

The high blood flow rate machine 36 may comprise an own control unit (not shown) connected to the pump 38 and to the ultrasound sensor and configured to control the pump 38. The pump 38, controlled by the respective control unit, is configured to generate in the blood circuit of the high blood flow rate machine 36 an average blood flow rate "Q2b" between 2 L/min and 5 L/min, which is much higher than the blood flow rate "$Q_b$" crossing the extracorporeal blood circuit of the apparatus 1.

The pump 38 is set against a pump speed and the high blood flow rate machine 36 comprises a flow meter, not shown, in the blood circuit to inform the control unit about the blood flow rate. The control unit adjusts the pump speed according to the blood flow rate from the flow meter. In other embodiments, the pump 38 may be controlled directly against the blood flow rate.

The apparatus 1 for extracorporeal blood treatment is combined with the high blood flow rate machine 36 by connecting the blood withdrawal line 6 and the blood return line 7 to the blood circuit of the high blood flow rate machine 36.

The blood circuit of the high blood flow rate machine 36 comprises (Figure 7B) a first connecting port 41 for connections to the connector 6 of the blood withdrawal line 6 and a second connecting port 42 for connections to the connector 7a of the blood return line 7. The connector and the respective port may be a Luer lock.

In the embodiment of Figures 3 and 7B, both the blood withdrawal line 6 and the blood return line 7 of the apparatus 1 are connected to the venous line 37 of the high blood flow rate machine 36. The blood withdrawal line 6 is connected between the pump 38 and the oxygenator 39, the blood return line 7 is connected upstream of the pump 38. According to a variant embodiment, not shown, the oxygenator 39 has an inlet port and an outlet port and the blood return line 7 of the apparatus 1 is connected to said outlet port and the blood withdrawal line 6 of the apparatus 1 is connected to said inlet port.

According to a variant embodiment (see the elements in dashed lines of Figures 3 and 7B), the blood withdrawal line 6 of the apparatus 1 is connected upstream of the pump 38, between the second connecting port 42 and the pump 38.

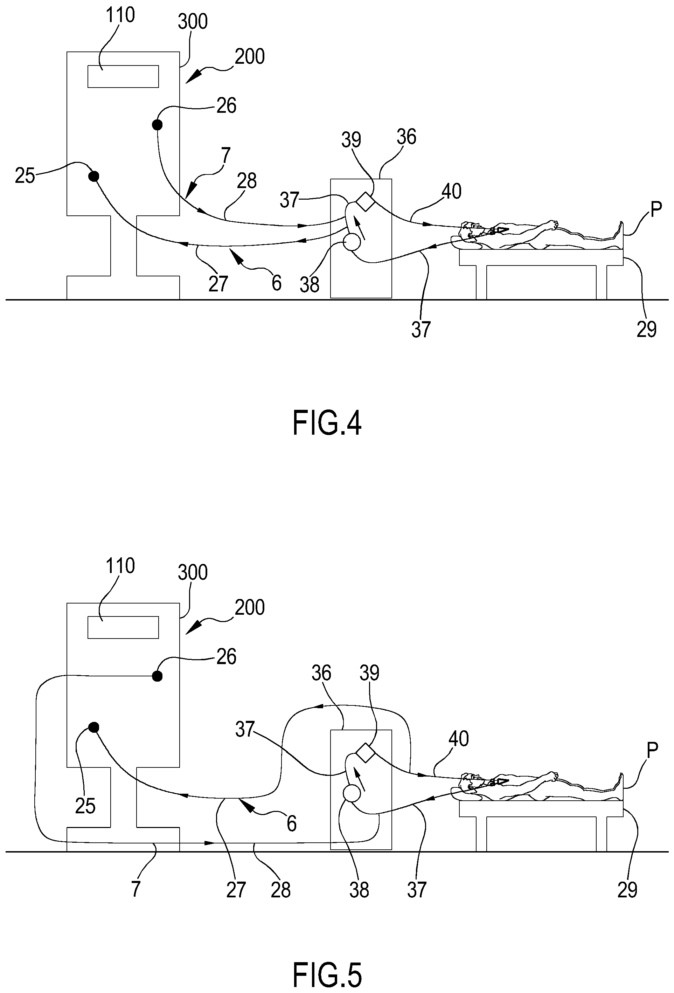

In the embodiment of Figure 4, both the blood withdrawal line 6 and the blood return line 7 of the apparatus 1 are connected to the venous line 37 of the high blood flow rate machine 36 between the pump 38 and the oxygenator 39. In the embodiment of Figure 5, the blood withdrawal line 6 of the apparatus 1 for extracorporeal blood treatment is connected to the oxygenated line 40 of the high blood flow rate machine 36 and the blood return line 7 of the apparatus 1 for extracorporeal blood treatment is connected to the venous line 37 of the high blood flow rate machine 36. A connection location of the blood return line 7 is positioned upstream of the pump 38 of the high blood flow rate machine 36.

In the embodiment of Figure 6, a connection location of the blood return line 7 of the apparatus 1 is positioned between the pump 38 and the oxygenator 39 of the high blood flow rate machine 36.

In other embodiments, other high blood flow rate machines 36 may be combined with the apparatus 1. For instance, the high blood flow rate machine 36 may be an Extracorporeal Life Support (ECLS) machine or a heart-lung machine for cardio-pulmonary by-pass (CPB).

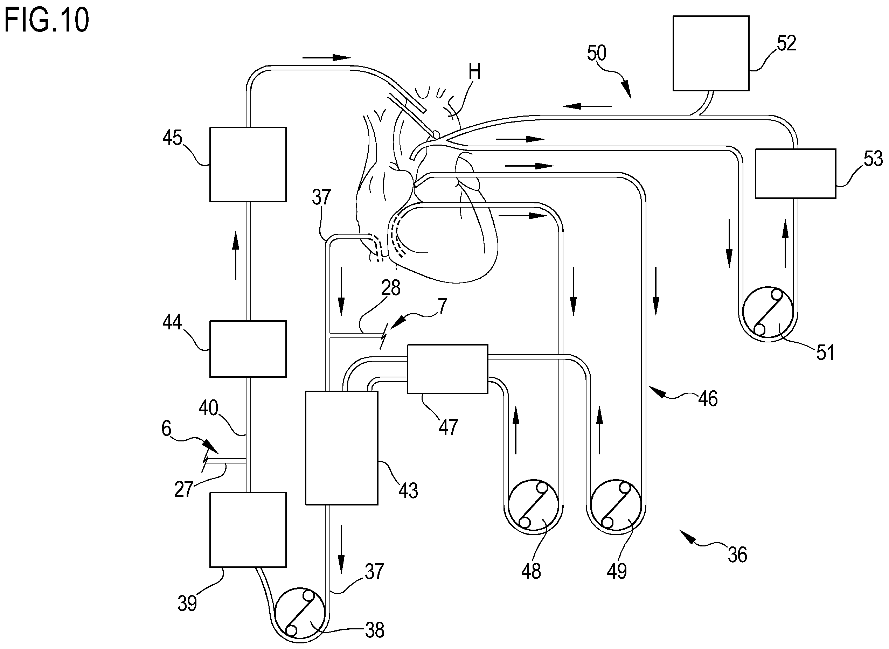

Figure 10 shows a cardio-pulmonary by-pass (CPB) or heart-lung machine, which is known per se. The cardio-pulmonary by-pass (CPB) comprises a venous line 37, a pump 38, an oxygenator 39, an oxygenated line 40 as the ECMO of the preceding Figures. In Figure 10, the venous line 37 is connected to the inferior vena cava of the heart H and the oxygenated line 40 is connected to the proximal aorta.

The cardio-pulmonary by-pass (CPB) further comprises: a venous reservoir 43 located along the venous line 37 and between the heart H and the pump 38; a heat exchanger 44 placed on the oxygenated line 40 and between the oxygenator 39 and the heart H; an arterial filter 45 placed between the heat exchanger 44 and the heart H. The cardio-pulmonary by-pass (CPB) further comprises a cardiotomy circuit 46 configured for suction during heart surgery. The cardiotomy circuit 46 comprises a cardiotomy reservoir 47 and cardiotomy blood pumps 48, 49. The cardio-pulmonary by-pass (CPB) further comprises a cardioplegia circuit 50 configured for delivering a cardioplegia fluid solution used to protect the heart H during cardio-pulmonary by-pass. The cardioplegia circuit 50 comprises a cardioplegia pump 51, a cardioplegia solution bag 52 and a cardioplegia heat exchanger 53 for cooling down the fluid solution.

As shown in Figure 10, the blood withdrawal line 6 of the apparatus 1 may be connected to the oxygenated line 40, between the oxygenator 39 and the heat exchanger 44. The blood return line 7 of the apparatus 1 may be connected to the venous line 37, between the heart H and the venous reservoir 43. Alternatively, the blood return line 7 may be connected to the venous reservoir 43.

**Detection of type of connection**

According to a method, not claimed, the control unit 100 of the apparatus 1 for extracorporeal blood treatment is configured and/or programmed for detecting if the extracorporeal blood circuit of the apparatus 1 is connected to the vascular access of the patient "P", like in Figures 1 and 2, or to the blood circuit of the high blood flow rate machine 36, like in Figures 3 to 6 and 10. If and when the type of connection has been detected, the control unit 100 issues one or more signals.

The identification of the type of connection may be used to automatically set working parameters of the apparatus 1 for extracorporeal blood treatment and/or to alert the operator if the manually entered type of connection is correct or not. Therefore, the issued signal may be a warning signal configured to alert the operator of the type of connection detected and/or the issued signal may be configured to automatically set the apparatus according to the type of connection detected.

For instance, after mechanical connection of the extracorporeal blood circuit to the vascular access device 400 or to the high blood flow rate machine 36, the operator, through the interface 110, enters the prescription parameters for the treatment and the type of connection (vascular access device 400 or high blood flow rate machine 36). The control unit 100 may also issue a query to the operator asking to identify and to enter the type of connection.

Then, the control unit 100 runs a routine, according to the algorithms discussed below, to identify the type of connection and may confirm to the operator that the type of connection is correct or warn the operator if the detected connection does not match with the one entered by the operator, so that the operator may change the setting of the apparatus 1.

In other embodiments, the control unit 100, once identified the type of connection, may automatically set the apparatus 1 according to the connection detected and/or may stop the apparatus if the detected connection does not match with the one entered by the operator.

The detection of the type of connection is based on gathering or measuring pressures from one or more pressure sensors in the extracorporeal blood circuit of the apparatus 1 and by performing an analysis of the gathered or measured pressure or pressures.

**Algorithm 1**

According to a first example algorithm, we consider an embodiment of the apparatus 1 in which the heights of the measurement locations of the access pressure sensor 25 and of the return pressure sensor 26 are the same and a hydrostatic bias is zero or may be ignored.

The high blood flow rate machine 36 (ECMO system) is a life supporting system it is assumed to be permanently running.

Before starting or re-starting a CRRT treatment through the apparatus 1 and while the blood pump 10 is not running and the blood flow rate "$Q_b$" is zero, the control unit 100 measures, through the access pressure sensor 25 and the return pressure sensor 26, a static access pressure "$P_{acc\_Qb0}$" in the blood withdrawal line 6 and a static return pressure "$P_{ret\_Qb0}$" in the blood return line 6.

Then, the control unit 100 compares the static return pressure "$P_{ret\_Qb0}$" with a lower limit pressure "$Lvasc_{min}$" and an upper limit pressure "$Lvasc_{max}$":

if

$$P_{ret\_Qb0} <= Lvasc_{min} \text{ or } P_{ret\_Qb0} >= Lvasc_{max}$$

then the control unit 100 records that the extracorporeal blood circuit of the apparatus 1 is connected to the blood circuit of the high blood flow rate machine 36;

if

$$Lvasc_{min} < P_{ret\_Qb0} < Lvasc_{max}$$

then the control unit 100 checks if the static access pressure "$P_{acc\_Qb0}$" is the same as the static return pressure

"$P_{ret\_Qb0}$" by computing a difference ($P_{acc\_Qb0}$ - $P_{ret\_Qb0}$) between the static access pressure "$P_{acc\_Qb0}$" and the static return pressure "$P_{ret\_Qb0}$" and comparing said difference with a lower error "-E" and an upper error "+E" ("+E and -E" are adopted to take into account measurement inaccuracies):

if

$$(P_{acc\_Qb0} - P_{ret\_Qb0}) <= -E$$

or

$$(P_{acc\_Qb0} - P_{ret\_Qb0}) >= +E$$

then the control unit 100 records that the extracorporeal blood circuit of the apparatus 1 is connected to the blood circuit of the high blood flow rate machine 36;
if

$$-E < (P_{acc\_Qb0} - P_{ret\_Qb0}) < +E$$

then the control unit 100 records that the extracorporeal blood circuit of the apparatus 1 is connected to the vascular access of the patient "P".

This first example algorithm compares the return pressure "$P_{ret\_Qb0}$" with the lower limit pressure "$Lvasc_{min}$" and the upper limit pressure "$Lvasc_{max}$". Anyway, also the access pressure "$P_{acc\_Qb0}$" may be used, depending on which pressure measurement is more accurate and robust for the apparatus 1.

The lower limit pressure "$Lvasc_{min}$" and the upper limit pressure "$Lvasc_{max}$" for considering offset pressure as compatible with vascular blood access are dependent on the apparatus (e.g. pressure measurement locations and measurement accuracy), on the venous central pressure of the patient "P" and on the position of patient "P". These limits may be set as $Lvasc_{min}$ = -20 mmHg and $Lvasc_{max}$ = +10 mmHg in an apparatus where the height of the pressure measurement locations is about 120 cm. An accepted error "E" when comparing the static access pressure and the static return pressure may be about 15 mmHg.

According to a variant of the first example algorithm, the hydrostatic bias $P_{hydr}$ due to different heights of the pressure measurement locations of the access pressure sensor 25 and return pressure sensor 26 is to be taken into account. In this variant, the lower limit pressure "$Lvasc_{min}$" and the upper limit pressure "$Lvasc_{max}$" referred to the static return pressure "$P_{ret\_Qb0}$" may be different from the limit pressures referred to the static access pressure "$P_{acc\_Qb0}$".

The static return pressure "$P_{ret\_Qb0}$" is compared with a return lower limit pressure and with a return upper limit pressure and the static access pressure "$P_{acc\_Qb0}$" is compared with an access lower limit pressure and with an access upper limit pressure.

If $Lretvasc_{min} < P_{ret\_Qb0} < Lretvasc_{max}$ and/or $Laccvasc_{min} < P_{ret\_Qb0} < Laccvasc_{max}$, then the control unit 100 computes the difference (($P_{acc\_Qb0}$ - $P_{ret\_Qb0}$) - $P_{hydr}$) and compares this difference with the lower error "-E" and the upper error "+E" as shown above.

**Algorithm 2**

According to a second example algorithm, the control unit 100 computes an access catheter pressure drop coefficient "$K_{cath}^{acc}$" and a return catheter pressure drop coefficient "$K_{cath}^{ret}$" through the following equations:

$$Eq.1)\quad K_{cath}^{ret} = ((P_{ret} - P_{ret\_Qb0})/(\mu \times Qb)) - K_{line}^{ret}$$

$$Eq.2)\quad K_{cath}^{acc} = ((P_{acc} - P_{acc\_Qb0})/(\mu \times Qb)) - K_{line}^{acc}$$

wherein

| | |
|---|---|
| Qb | measured or set blood flow rate steady and different from zero; |
| $\mu$ | blood viscosity; |
| $P_{ret}$, $P_{acc}$ | measured return and access pressures when the blood flow rate is steady and different from zero; |
| $P_{ret\_Qb0}$, $P_{acc\_Qb0}$ | measured return and access pressures when the blood flow rate is zero; |
| $K_{line}^{ret}$, $K_{line}^{acc}$ | pressure drop coefficients of the blood return line 7 and blood withdrawal line 6. |

The blood viscosity and the pressure drop coefficients of the blood return line 7 and blood withdrawal line 6 may be derived from experimental testing or computed through the following equations:

$$\text{Eq.3)} \quad \mu = e^{(1{,}8/T) \times 5{,}54} \times e^{2{,}3 \times (Hct/100)}$$

$\mu$     blood viscosity (mPa.s);
Hct     blood hematocrit (percent [0-100]);
T     blood temperature (°C).

$$\text{Eq.4)} \quad K_{line}^{ret} = (128 \times L_{ret})/(\pi \times d_{ret}^{4}) \ (mmHg/(ml/min)/(mPa \ s))$$

$$\text{Eq.5)} \quad K_{line}^{acc} = (128 \times L_{acc})/(\pi \times d_{acc}^{4}) \ (mmHg/(ml/min)/(mPa \ s))$$

$L_{ret}, d_{ret}$     length and the internal diameter of the section 28 of the blood return line 7;
$L_{acc}, d_{acc}$     length and the internal diameter of the section 27 of the blood withdrawal line 6.

Then, the control unit 100 compares the pressure drop coefficients "$K_{cath}^{ret}$", "$K_{cath}^{acc}$" of the blood return line 7 and blood withdrawal line 6 with a pressure drop coefficient reference value "$K_{cath}^{min}$":

if

$$K_{cath}^{ret} < K_{cath}^{min} \ \text{and} \ K_{cath}^{acc} < K_{cath}^{min}$$

then the control unit 100 records that the extracorporeal blood circuit of the apparatus 1 is connected to the blood circuit of the high blood flow rate machine 36;

if

$$K_{cath}^{ret} >= K_{cath}^{min} \ \text{and} \ K_{cath}^{acc} >= K_{cath}^{min}$$

then the control unit 100 records that the extracorporeal blood circuit of the apparatus 1 is connected to the vascular access of the patient "P";

if

$$K_{cath}^{ret} >= K_{cath}^{min} \ \text{and} \ K_{cath}^{acc} < K_{cath}^{min} \ \text{or}$$

if

$$K_{cath}^{ret} < K_{cath}^{min} \ \text{and} \ K_{cath}^{acc} >= K_{cath}^{min}$$

then the control unit records a status of uncertain connection.

The value of "$K_{cath}^{min}$" may be derived from an analysis of pressure drop of the catheters of each specific apparatus or derived from an analysis of clinical records.

These decision steps acknowledges that, in the case of high blood flow rate machine blood access, both access and return lines are directly connected to the high flow blood circuit and that the expected outcome of Equations 1 and 2 is a zero pressure drop coefficient, as no catheter is present at the end of the blood lines.

**Algorithm 3**

Checking for the correlation between access pressure "$P_{acc}$" and return pressures "$P_{ret}$" may be a third way to diagnose between vascular access through a catheter and connection to the high blood flow rate machine 36. This third way is performed during the CRRT treatment and while the blood pump 10 is running and the blood flow rate "$Q_b$" is other than zero.

According to this third way, the access pressure "$P_{acc}$" and the return pressure "$P_{ret}$" are measured and monitored over time during the extracorporeal blood treatment while the blood flow rate "$Q_b$" is kept constant.

The following example is valid only in the circumstance where Pacc <0 and Pret>0, that may match with access to catheter or to blood circuit of a high blood flow rate machine.

If, at some point of time, the access pressure "$P_{acc}$" decreases and the return pressure "$P_{ret}$" increases, then the control unit records that the extracorporeal blood circuit is connected to the blood circuit of the high blood flow rate machine 36. This is the scenario where blood flow in the high blood flow rate machine 36 increases.

Also if, at some point of time, the access pressure "$P_{acc}$" increases and the return pressure "$P_{ret}$" decreases, then the control unit records that the extracorporeal blood circuit is connected to the blood circuit of the high blood flow rate machine 36. This is the symmetrical scenario where blood flow in the high blood flow rate machine 36 decreases. Similar algorithm may be developed for other configuration of connections.

Algorithms 1, 2 and 3 may also be implemented as a two or three steps diagnostic method. For instance, algorithms 1 and 2 may be applied in sequence. Algorithm 3 may be used to confirm an initial main diagnostic performed using algorithm 1 and/or 2, if the main diagnostic step provides a status of uncertain connection, e.g. if pressure conditions are in a range compatible both with the vascular access of the patient "P" and with the connection to the blood circuit of the high blood flow rate machine 36.

Once the type of connection has been identified, the control unit 100 may automatically set the apparatus 1 according to the type of connection detected or may alert the operator who, through the interface 110, sets the apparatus 1 according to the type of connection detected. The following Table 1 discloses, by way of example, operating pressures which are accepted according to the connection detected (vascular access connection or ECMO connection).

Table 1

| Pressure sensor | Vascular access | ECMO |
|---|---|---|
| Return | -20 mmHg to +350 mmHg | -250 mmHg to +400 mmHg |
| Access | -250 mmHg to +20 mmHg | -250 mmHg to +400 mmHg |

As shown in Table 1, during combined working of the apparatus for extracorporeal blood treatment and ECMO, return pressure operating range is expanded to allow operation at negative pressure that is present before the high flow centrifugal pump 38 in the ECMO circuit and to support operation at higher positive pressures. The access pressure has been adjusted to allow operation at positive pressure that is present after the high flow centrifugal pump 38 in the ECMO circuit.

The following Tables 2 and 3 disclose, by way of example, alarm pressures and limits which are set according to the connection detected (vascular access or ECMO). The pressure monitoring system of the apparatus 1 is updated to allow operating at negative access/return pressures.

Table 2 - Return obstruction alarm

| Access configuration | Vascular access | ECMO |
|---|---|---|
| Initial operating pressure | -20 to +350 | -250 to +400 |
| Alarm threshold | P > +350 mmHg | P > 400 mmHg OR P increase > 200 mmHg |

Table 3 - Access obstruction alarm

| Access configuration | Vascular access | ECMO |
|---|---|---|
| Initial operating pressure | -250 to +20 | -250 to +400 |
| Alarm threshold | P < -250 | P < -250 OR P decrease > 200 mmHg |

In the above tables, the pressure increase/decrease terms relate to fast changes in the related pressure, e.g. pressure shifts occurring in less than 5 seconds, optionally less than 2 seconds.

If the blood warming device "W" or the fluid warmer is/are present, the control unit 100 automatically enables the blood warming device "W" and/or the fluid warmer if the vascular access is connected or disables the blood warming device "W" and/or the fluid warmer if the ECMO circuit is connected.

Indeed, the apparatus 1 for extracorporeal blood treatment should comply with requirements limiting the current leakages and the connection of the apparatus 1 with the ECMO may make the situation worse. Since the blood warming devices "W" and the fluid warmers accounts for a great part of current leakages, it is better to prevent use of these warmers when combining the apparatus 1 with the ECMO.

Furthermore, since the ECMO controls the temperature of the patient "P" with much more impact than the apparatus 1 for extracorporeal blood treatment, warming operated by the blood warming device "W" in the apparatus 1 may be useless.

While the invention has been described in connection with what is presently considered to be the most practical and preferred embodiment, it is to be understood that the invention is not to be limited to the disclosed embodiment, but on the contrary, is intended to cover various modifications and equivalent arrangements included within the scope of the appended claims.

## Claims

1. Apparatus for extracorporeal blood treatment, comprising:

    a treatment unit (2);
    an extracorporeal blood circuit having a blood withdrawal line (6) connected to an inlet of the treatment unit (2) and a blood return line (7) connected to an outlet of the treatment unit (2); said extracorporeal blood circuit being configured for connection either to a vascular access of a patient (P) or for connection to a blood circuit of a high blood flow rate machine (36), the high blood flow rate machine (36) being an Extra Corporeal Membrane Oxygenation (ECMO) machine or an Extracorporeal Life Support (ECLS) machine or a heart-lung machine for cardio-pulmonary by-pass;
    at least one pressure sensor (25, 26) configured to detect a pressure in at least one measurement location in the extracorporeal blood circuit;
    a blood pump (10) configured to control a flow of blood (Qb) through the extracorporeal blood circuit;
    a control unit (100) connected to the blood pump (10) and to the at least one pressure sensor (25, 26), the control unit (100) being configured for checking a type of connection of the extracorporeal blood circuit by performing the following procedure:

        - receiving at least one measured pressure from the at least one pressure sensor (25, 26);

    **characterized by** the fact that the procedure further includes:

        - detecting if the extracorporeal blood circuit is connected to the vascular access of the patient (P) or to the blood circuit of the high blood flow rate machine (36) by performing an analysis of the at least one measured pressure or of at least one parameter correlated to the at least one measured pressure, the step of detecting comprising an automatic identification of the type of connection between a connection of the extracorporeal blood circuit to the vascular access of the patient and a connection of the extracorporeal blood circuit to the blood circuit of the high blood flow rate machine (36);
        - issuing at least one signal if or when the connection to the vascular access of the patient (P) and/or the connection to the blood circuit of the high blood flow rate machine (36) is detected.

2. The apparatus of claim 1, wherein the at least one measured pressure comprises an access pressure ($P_{acc}$, $P_{acc\_Qb0}$) and/or a return pressure ($P_{ret}$, $P_{ret\_Qb0}$);
    wherein the at least one pressure sensor (25, 26) comprises:

    an access pressure sensor (25) configured to measure the access pressure ($P_{acc}$, $P_{acc\_Qb0}$) at a measurement location in the blood withdrawal line (6); and/or
    a return pressure sensor (26) configured to measure the return pressure ($P_{ret}$, $P_{ret\_Qb0}$) at a measurement location in the blood return line (7).

3. The apparatus of claim 1 or 2, wherein performing the analysis comprises: comparing the at least one measured pressure or the at least one parameter correlated to the at least one measured pressure with at least one reference value.

4. The apparatus of claim 3, wherein the at least one measured pressure comprises a static pressure ($P_{ret\_Qb0}$, $P_{acc\_Qb0}$) measured by the at least one pressure sensor (25, 26) when the flow of blood (Qb) through the extracorporeal blood circuit is zero and wherein the at least one reference value comprises a lower limit pressure ($Lvasc_{min}$) and an upper limit pressure ($Lvasc_{max}$);

wherein the control unit (100) is configured for performing the following procedure:

receiving the static pressure ($P_{ret\_Qb0}$, $P_{acc\_Qb0}$);
receiving the lower limit pressure ($Lvasc_{min}$) and the upper limit pressure ($Lvasc_{max}$);
comparing the static pressure ($P_{ret\_Qb0}$, $P_{acc\_Qb0}$) with the lower limit pressure ($Lvasc_{min}$) and with the upper limit pressure ($Lvasc_{max}$);

wherein:
if the static pressure ($P_{ret\_Qb0}$, $P_{acc\_Qb0}$) is outside a range between the lower limit pressure ($Lvasc_{min}$) and the upper limit pressure ($Lvasc_{max}$), then the control unit (100) records that the extracorporeal blood circuit is connected to the blood circuit of the high blood flow rate machine (36).

5. The apparatus of claim 3 when depending on claim 2, wherein the at least one measured pressure ($P_{ret,}$ $P_{ret\_Qb0}$, $P_{acc,}$ $P_{acc\_Qb0}$) comprises a static return pressure ($P_{ret\_Qb0}$) and a static access pressure ($P_{acc-Qb0}$) measured by the access pressure sensor (25) and by the return pressure sensor (26) when the flow of blood (Qb) through the extracorporeal blood circuit is zero, wherein the at least one reference value comprises at least one lower limit pressure ($Lvasc_{min}$) and at least one upper limit pressure ($Lvasc_{max}$);
wherein the control unit (100) is configured for performing the following procedure:

receiving the static return pressure ($P_{ret\_Qb0}$) and the static access pressure ($P_{acc\_Qb0}$);
receiving the at least one lower limit pressure ($Lvasc_{min}$) and the at least one upper limit pressure ($Lvasc_{max}$);
comparing the static return pressure ($P_{ret\_Qb0}$) and/or the static access pressure ($P_{acc-Qb0}$) with the at least one lower limit pressure ($Lvasc_{min}$) and with the at least one upper limit pressure ($Lvasc_{max}$);
wherein:
if the static return pressure ($P_{ret\_Qb0}$) and/or the static access pressure ($P_{acc-Qb0}$) is outside a range between the at least one lower limit pressure ($Lvasc_{min}$) and the at least one upper limit pressure ($Lvasc_{max}$), then the control unit (100) records that the extracorporeal blood circuit is connected to the blood circuit of the high blood flow rate machine (36).

6. The apparatus of claim 5, wherein if the static return pressure ($P_{ret\_Qb0}$) and/or the static access pressure ($P_{acc\_Qb0}$) is within the range between the at least one lower limit pressure ($Lvasc_{min}$) and the at least one upper limit pressure ($Lvasc_{max}$), then the control unit (100) checks if: a difference between the static access pressure ($P_{acc-Qb0}$) and the static return pressure ($P_{ret\_Qb0}$) is null in case the heights of a measurement location of the access pressure performed through the access pressure sensor (25) and of a measurement location of the return pressure performed through the return pressure sensor (26) are the same; or a difference between the static access pressure ($P_{acc-Qb0}$) and the static return pressure ($P_{ret\_Qb0}$) is equal to an hydrostatic bias ($P_{hydr}$) due to different heights of the measurement location of the access pressure performed through the access pressure sensor (25) and of the measurement location of the return pressure performed through the return pressure sensor (26);
wherein:

if the difference between the static access pressure ($P_{acc\_Qb0}$) and the static return pressure ($P_{ret\_Qb0}$) is not null or if the difference between the static access pressure ($P_{acc\_Qb0}$) and the static return pressure ($P_{ret\_Qb0}$) is not equal to the hydrostatic bias ($P_{hydr}$), then the control unit (100) records that the extracorporeal blood circuit is connected to the blood circuit of the high blood flow rate machine (36); or
if the difference between the static access pressure ($P_{acc\_Qb0}$) and the static return pressure ($P_{ret\_Qb0}$) is null or if the difference between the static access pressure ($P_{acc\_Qb0}$) and the static return pressure ($P_{ret\_Qb0}$) is equal to the hydrostatic bias ($P_{hydr}$), then the control unit (100) records that the extracorporeal blood circuit is connected to the vascular access of the patient (P).

7. The apparatus of claim 3, wherein the at least one parameter correlated to the at least one measured pressure comprises at least one catheter pressure drop coefficient ($K_{cath}$) and wherein the at least one reference value comprises a pressure drop coefficient reference value ($K_{cath\_min}$);
wherein the control unit (100) is configured for performing the following procedure:

receiving the pressure drop coefficient reference value ($K_{cath-min}$);

calculating the at least one catheter pressure drop coefficient ($K_{cath}$);

wherein:

if the at least one catheter pressure drop coefficient ($K_{cath}$) is lower than the pressure drop coefficient reference value ($K_{cath\_min}$), then the control unit (100) records that the extracorporeal blood circuit is connected to the blood circuit of the high blood flow rate machine (36);

if the at least one catheter pressure drop coefficient ($K_{cath}$) is equal to or greater than the pressure drop coefficient reference value ($K_{cath\_min}$) then the control unit (100) records that the extracorporeal blood circuit is connected to the vascular access of the patient (P).

8. The apparatus of claim 7, wherein the at least one catheter pressure drop coefficient ($K_{cath}$) comprises an access catheter pressure drop coefficient ($K_{cath}^{acc}$) and/or a return catheter pressure drop coefficient ($K_{cath}^{ret}$); wherein:

if the access catheter pressure drop coefficient ($K_{cath}^{acc}$) and the return catheter pressure drop coefficient ($K_{cath}^{ret}$) are lower than the pressure drop coefficient reference value ($K_{cath\_min}$), then the control unit (100) records that the extracorporeal blood circuit is connected to the blood circuit of the high blood flow rate machine (36); or

if the access catheter pressure drop coefficient ($K_{cath}^{acc}$) and the return catheter pressure drop coefficient ($K_{cath}^{ret}$) are equal to or greater than the pressure drop coefficient reference value ($K_{cath\_min}$), then the control unit (100) records that the extracorporeal blood circuit is connected to the vascular access of the patient (P).

9. The apparatus of claim 8, wherein, if the access catheter pressure drop coefficient ($K_{cath}^{acc}$) is equal to or greater than the pressure drop coefficient reference value ($K_{cath\_min}$) and the return catheter pressure drop coefficient ($K_{cath}^{ret}$) is lower than the pressure drop coefficient reference value ($K_{cath\_min}$) or vice versa, then the control unit (100) records a status of uncertain connection.

10. The apparatus of claim 2, wherein performing the analysis comprises: checking a correlation between the access pressure ($P_{acc}$) and the return pressure ($P_{ret}$).

11. The apparatus of claim 10, wherein checking a correlation comprises monitoring the access pressure ($P_{acc}$) and the return pressure ($P_{ret}$) over time during at least part of the extracorporeal blood treatment; wherein if the access pressure ($P_{acc}$) decreases and the return pressure ($P_{ret}$) increases or the access pressure increases ($P_{acc}$) and the return pressure decreases ($P_{ret}$) while the blood flow rate (Qb) through the extracorporeal blood circuit is kept constant, then the control unit (100) records that the extracorporeal blood circuit is connected to the blood circuit of the high blood flow rate machine (36).

12. The apparatus of any of claims 1 to 11, wherein the control unit (100) is configured to check the type of connection at the start or re-start of an extracorporeal blood treatment and/or along the extracorporeal blood treatment.

13. The apparatus of any of claims 1 to 12, wherein issuing at least one signal comprises:

emitting a warning signal configured to alert the operator of the type of connection detected; and/or

notify to the operator working parameters according to the type of connection detected; and/or

setting the apparatus with working parameters according to the type of connection detected.

14. A combination of an apparatus for extracorporeal blood treatment and a high blood flow rate machine; wherein the apparatus for extracorporeal blood treatment is according to any of the preceding claims 1 to 13; wherein the high blood flow rate machine is an Extra Corporeal Membrane Oxygenation (ECMO) machine or an Extracorporeal Life Support (ECLS) machine or a heart-lung machine for cardio-pulmonary by-pass, and wherein the apparatus (1) for extracorporeal blood treatment is a continuous renal replacement therapy (CRRT) apparatus for intensive care treatments.

15. The combination of the preceding claim 14, wherein the high blood flow rate machine (36) comprises:

a blood circuit comprising a venous line (37) and an oxygenated line (40) provided with cannulae configured to be placed in veins or arteries of a patient (P);

a pump (38);

an oxygenator (39);
wherein the pump (38) is configured to pump blood through the blood circuit and through the oxygenator (39) with a blood flow rate greater than 1 L/min, optionally greater than 2 L/min, optionally between 2 L/min and 5 L/min; wherein the blood withdrawal line (6) and the blood return line (7) of the apparatus (1) for extracorporeal blood treatment are connected/able to the venous line (37) and/or to the oxygenated line (40) of the high blood flow rate machine (36).

**Patentansprüche**

1. Vorrichtung zur extrakorporalen Blutbehandlung, umfassend:

    eine Behandlungseinheit (2);
    einen extrakorporalen Blutkreislauf mit einer Blutentnahmeleitung (6), die mit einem Einlass der Behandlungseinheit (2) verbunden ist, und einer Blutrückführleitung (7), die mit einem Auslass der Behandlungseinheit (2) verbunden ist; wobei der extrakorporale Blutkreislauf zur Verbindung mit entweder einem Gefäßzugang eines Patienten (P) oder zur Verbindung mit einem Blutkreislauf einer Maschine mit hoher Blutflussrate (36) gestaltet ist, wobei die Maschine mit hoher Blutflussrate (36) eine extrakorporale Membranoxygenierungs(ECMO)-Maschine oder eine extrakorporale Lebenserhaltungs(ECLS)-Maschine oder eine Herz-Lungen-Maschine für kardiopulmonalen Bypass ist;
    wenigstens einen Drucksensor (25, 26), gestaltet zum Erfassen eines Drucks an wenigstens einer Messstelle in dem extrakorporalen Blutkreislauf;
    eine Blutpumpe (10), gestaltet zum Steuern eines Blutflusses (Qb) durch den extrakorporalen Blutkreislauf;
    eine Steuereinheit (100), die mit der Blutpumpe (10) und mit dem wenigstens einen Drucksensor (25, 26) verbunden ist, wobei die Steuereinheit (100) dafür gestaltet ist, eine Art der Verbindung des extrakorporalen Blutkreislaufs durch Durchführen des folgenden Verfahrens zu überprüfen:

    - Empfangen wenigstens eines gemessenen Drucks von dem wenigstens einen Drucksensor (25, 26);

    **dadurch gekennzeichnet, dass** das Verfahren ferner umfasst:

    - Erfassen, ob der extrakorporale Blutkreislauf mit dem Gefäßzugang des Patienten (P) oder mit dem Blutkreislauf der Maschine mit hoher Blutflussrate (36) verbunden ist, durch Durchführen einer Analyse des wenigstens einen gemessenen Drucks oder wenigstens eines Parameters, der mit dem wenigstens einen gemessenen Druck korreliert ist, wobei der Schritt des Erfassens eine automatische Identifizierung der Art der Verbindung zwischen einer Verbindung des extrakorporalen Blutkreislaufs mit dem Gefäßzugang des Patienten und einer Verbindung des extrakorporalen Blutkreislaufs mit dem Blutkreislauf der Maschine mit hoher Blutflussrate (36) umfasst;
    - Ausgeben wenigstens eines Signals, wenn die Verbindung mit dem Gefäßzugang des Patienten (P) und/oder die Verbindung mit dem Blutkreislauf der Maschine mit hoher Blutflussrate (36) erfasst wird.

2. Vorrichtung nach Anspruch 1, wobei der wenigstens eine gemessene Druck einen Zugangsdruck ($P_{acc}$, $P_{acc\_Qb0}$) und/oder einen Rücklaufdruck ($P_{ret}$, $P_{ret\_Qb0}$) umfasst; wobei der wenigstens eine Drucksensor (25, 26) umfasst:

    einen Zugangsdrucksensor (25), gestaltet zum Messen des Zugangsdrucks ($P_{acc}$, $P_{acc\_Qb0}$) an einer Messstelle in der Blutentnahmeleitung (6); und/oder
    einen Rücklaufdrucksensor (26), gestaltet zum Messen des Rücklaufdrucks ($P_{ret}$, $P_{ret\_Qb0}$) an einer Messstelle in der Blutrückführleitung (7).

3. Vorrichtung nach Anspruch 1 oder 2, wobei Durchführen der Analyse umfasst: Vergleichen des wenigstens einen gemessenen Drucks oder des wenigstens einen Parameters, der mit dem wenigstens einen gemessenen Druck korreliert ist, mit wenigstens einem Referenzwert.

4. Vorrichtung nach Anspruch 3, wobei der wenigstens eine gemessene Druck einen statischen Druck ($P_{ret\_Qb0}$, $P_{acc\_Qb0}$) umfasst, der durch den wenigstens einen Drucksensor (25, 26) gemessen wird, wenn der Blutfluss (Qb) durch den extrakorporalen Blutkreislauf null ist, und wobei der wenigstens eine Referenzwert einen unteren Grenzdruck ($Lvasc_{min}$) und einen oberen Grenzdruck ($Lvasc_{max}$) umfasst; wobei die Steuereinheit (100) gestaltet ist zum Durchführen des folgenden Verfahrens:

Empfangen des statischen Drucks ($P_{ret\_Qb0}$, $P_{acc\_Qb0}$);
Empfangen des unteren Grenzdrucks ($Lvasc_{min}$) und des oberen Grenzdrucks ($Lvasc_{max}$);
Vergleichen des statischen Drucks ($P_{ret\_Qb0}$, $P_{acc\_Qb0}$) mit dem unteren Grenzdruck ($Lvasc_{min}$) und mit dem oberen Grenzdruck ($Lvasc_{max}$);
wobei:
wenn der statische Druck ($P_{ret\_Qb0}$, $P_{acc\_Qb0}$) außerhalb eines Bereichs zwischen dem unteren Grenzdruck ($Lvasc_{min}$) und dem oberen Grenzdruck ($Lvasc_{max}$) liegt, die Steuereinheit (100) festhält, dass der extrakorporale Blutkreislauf mit dem Blutkreislauf der Maschine mit hoher Blutflussrate (36) verbunden ist.

5. Vorrichtung nach Anspruch 3, wenn abhängig von Anspruch 2, wobei der wenigstens eine gemessene Druck ($P_{ret}$, $P_{ret\_Qb0}$, $P_{acc}$, $P_{acc\_Qb0}$) einen statischen Rücklaufdruck ($P_{ret\_Qb0}$), der durch den Rücklaufdrucksensor (26) gemessen wird, und einen statischen Zugangsdruck ($P_{acc\_Qb0}$), der durch den Zugangsdrucksensor (25) gemessen wird, umfasst, wenn der Blutfluss (Qb) durch den extrakorporalen Blutkreislauf null ist, wobei der wenigstens eine Referenzwert wenigstens einen unteren Grenzdruck ($Lvasc_{min}$) und wenigstens einen oberen Grenzdruck ($Lvasc_{max}$) umfasst;
wobei die Steuereinheit (100) gestaltet ist zum Durchführen des folgenden Verfahrens:

Empfangen des statischen Rücklaufdrucks ($P_{ret\_Qb0}$) und des statischen Zugangsdrucks ($P_{acc\_Qb0}$) ;
Empfangen des wenigstens einen unteren Grenzdrucks ($Lvasc_{min}$) und des wenigstens einen oberen Grenzdrucks ($Lvasc_{max}$) ;
Vergleichen des statischen Rücklaufdrucks ($P_{ret\_Qb0}$) und/oder des statischen Zugangsdrucks ($P_{acc\_Qb0}$) mit dem wenigstens einen unteren Grenzdruck ($Lvasc_{min}$) und mit dem wenigstens einen oberen Grenzdruck ($Lvasc_{max}$);
wobei:
wenn der statische Rücklaufdruck ($P_{ret\_Qb0}$) und/oder der statische Zugangsdruck ($P_{acc\_Qb0}$) außerhalb eines Bereichs zwischen dem wenigstens einen unteren Grenzdruck ($Lvasc_{min}$) und dem wenigstens einen oberen Grenzdruck ($Lvasc_{max}$) liegen, die Steuereinheit (100) festhält, dass der extrakorporale Blutkreislauf mit dem Blutkreislauf der Maschine mit hoher Blutflussrate (36) verbunden ist.

6. Vorrichtung nach Anspruch 5, wobei, wenn der statische Rücklaufdruck ($P_{ret\_Qb0}$) und/oder der statische Zugangsdruck ($P_{acc\_Qb0}$) innerhalb des Bereichs zwischen dem wenigstens einen unteren Grenzdruck ($Lvasc_{min}$) und dem wenigstens einen oberen Grenzdruck ($Lvasc_{max}$) liegen, die Steuereinheit (100) prüft, ob: eine Differenz zwischen dem statischen Zugangsdruck ($P_{acc\_Qb0}$) und dem statischen Rücklaufdruck ($P_{ret\_Qb0}$) null ist, falls die Höhen einer Messstelle des Zugangsdrucks, durchgeführt durch den Zugangsdrucksensor (25), und einer Messstelle des Rücklaufdrucks, durchgeführt durch den Rücklaufdrucksensor (26), gleich sind; oder eine Differenz zwischen dem statischen Zugangsdruck ($P_{acc\_Qb0}$) und dem statischen Rücklaufdruck ($P_{ret\_Qb0}$) aufgrund unterschiedlicher Höhen der Messstelle des Zugangsdrucks, durchgeführt durch den Zugangsdrucksensor (25), und der Messstelle des Rücklaufdrucks, durchgeführt durch den Rücklaufdrucksensor (26), gleich einem hydrostatischen Offset ($P_{hydr}$) ist;
wobei:

wenn die Differenz zwischen dem statischen Zugangsdruck ($P_{acc\_Qb0}$) und dem statischen Rücklaufdruck ($P_{ret\_Qb0}$) nicht null ist oder wenn die Differenz zwischen dem statischen Zugangsdruck ($P_{acc\_Qb0}$) und dem statischen Rücklaufdruck ($P_{ret\_Qb0}$) nicht gleich dem hydrostatischen Offset ($P_{hydr}$) ist, die Steuereinheit (100) festhält, dass der extrakorporale Blutkreislauf mit dem Blutkreislauf der Maschine mit hoher Blutflussrate (36) verbunden ist; oder
wenn die Differenz zwischen dem statischen Zugangsdruck ($P_{acc\_Qb0}$) und dem statischen Rücklaufdruck ($P_{ret\_Qb0}$) null ist oder wenn die Differenz zwischen dem statischen Zugangsdruck ($P_{acc\_Qb0}$) und dem statischen Rücklaufdruck ($P_{ret\_Qb0}$) gleich dem hydrostatischen Offset ($P_{hydr}$) ist, die Steuereinheit (100) festhält, dass der extrakorporale Blutkreislauf mit dem Gefäßzugang des Patienten (P) verbunden ist.

7. Vorrichtung nach Anspruch 3, wobei der wenigstens eine Parameter, der mit dem wenigstens einen gemessenen Druck korreliert ist, wenigstens einen Katheter-Druckabfallkoeffizienten ($K_{cath}$) umfasst und wobei der wenigstens eine Referenzwert einen Druckabfallkoeffizient-Referenzwert ($K_{cath\_min}$) umfasst; wobei die Steuereinheit (100) gestaltet ist zum Durchführen des folgenden Verfahrens:

Empfangen des Druckabfallkoeffizient-Referenzwerts ($K_{cath\_min}$) ;
Berechnen des wenigstens einen Katheter-Druckabfallkoeffizienten ($K_{cath}$) ;

wobei:

wenn der wenigstens eine Katheter-Druckabfallkoeffizient ($K_{cath}$) kleiner als der Druckabfallkoeffizient-Referenzwert ($K_{cath\_min}$) ist, die Steuereinheit (100) festhält, dass der extrakorporale Blutkreislauf mit dem Blutkreislauf der Maschine mit hoher Blutflussrate (36) verbunden ist;

wenn der wenigstens eine Katheter-Druckabfallkoeffizient ($K_{cath}$) gleich oder größer als der Druckabfallkoeffizient-Referenzwert ($K_{cath\_min}$) ist, die Steuereinheit (100) festhält, dass der extrakorporale Blutkreislauf mit dem Gefäßzugang des Patienten (P) verbunden ist.

8. Vorrichtung nach Anspruch 7, wobei der wenigstens eine Katheter-Druckabfallkoeffizient ($K_{cath}$) einen Zugangskatheter-Druckabfallkoeffizienten ($K_{cath}^{acc}$) und/oder einen Rückführkatheter-Druckabfallkoeffizienten ($K_{cath}^{ret}$) umfasst;

wobei:

wenn der Zugangskatheter-Druckabfallkoeffizient ($K_{cath}^{acc}$) und der Rückführkatheter-Druckabfallkoeffizient ($K_{cath}^{ret}$) kleiner als der Druckabfallkoeffizient-Referenzwert ($K_{cath\_min}$) sind, die Steuereinheit (100) festhält, dass der extrakorporale Blutkreislauf mit dem Blutkreislauf der Maschine mit hoher Blutflussrate (36) verbunden ist; oder

wenn der Zugangskatheter-Druckabfallkoeffizient ($K_{cath}^{acc}$) und der Rückführkatheter-Druckabfallkoeffizient ($K_{cath}^{ret}$) gleich oder größer als der Druckabfallkoeffizient-Referenzwert ($K_{cath\_min}$) sind, die Steuereinheit (100) festhält, dass der extrakorporale Blutkreislauf mit dem Gefäßzugang des Patienten (P) verbunden ist.

9. Vorrichtung nach Anspruch 8, wobei, wenn der Zugangskatheter-Druckabfallkoeffizient ($K_{cath}^{acc}$) gleich oder größer als der Druckabfallkoeffizient-Referenzwert ($K_{cath\_min}$) ist und der Rückführkatheter-Druckabfallkoeffizient ($K_{cath}^{ret}$) kleiner als der Druckabfallkoeffizient-Referenzwert ($K_{cath\_min}$) ist oder umgekehrt, die Steuereinheit (100) einen Status einer ungewissen Verbindung festhält.

10. Vorrichtung nach Anspruch 2, wobei Durchführen der Analyse umfasst: Überprüfen einer Korrelation zwischen dem Zugangsdruck ($P_{acc}$) und dem Rücklaufdruck ($P_{ret}$).

11. Vorrichtung nach Anspruch 10, wobei Überprüfen einer Korrelation Überwachen des Zugangsdrucks ($P_{acc}$) und des Rücklaufdrucks ($P_{ret}$) über die Zeit während wenigstens eines Teils der extrakorporalen Blutbehandlung umfasst; wobei, wenn der Zugangsdruck ($P_{acc}$) abnimmt und der Rücklaufdruck ($P_{ret}$) zunimmt oder der Zugangsdruck ($P_{acc}$) zunimmt und der Rücklaufdruck ($P_{ret}$) abnimmt, während die Blutflussrate ($Qb$) durch den extrakorporalen Blutkreislauf konstant gehalten wird, die Steuereinheit (100) festhält, dass der extrakorporale Blutkreislauf mit dem Blutkreislauf der Maschine mit hoher Blutflussrate (36) verbunden ist.

12. Vorrichtung nach einem der Ansprüche 1 bis 11, wobei die Steuereinheit (100) dafür gestaltet ist, die Art der Verbindung bei Beginn oder Neubeginn einer extrakorporalen Blutbehandlung und/oder während der extrakorporalen Blutbehandlung zu überprüfen.

13. Vorrichtung nach einem der Ansprüche 1 bis 12, wobei Ausgeben wenigstens eines Signals umfasst:

Aussenden eines Warnsignals, das dafür gestaltet ist, dem Bediener die erfasste Art der Verbindung zu melden; und/oder

Informieren des Bedieners über Arbeitsparameter gemäß der erfassten Art der Verbindung; und/oder

Einstellen der Vorrichtung mit Arbeitsparametern gemäß der erfassten Art der Verbindung.

14. Kombination einer Vorrichtung zur extrakorporalen Blutbehandlung und einer Maschine mit hoher Blutflussrate; wobei die Vorrichtung zur extrakorporalen Blutbehandlung nach einem der vorstehenden Ansprüche 1 bis 13 ist; wobei die Maschine mit hoher Blutflussrate eine extrakorporale Membranoxygenierungs(ECMO)-Maschine oder eine extrakorporale Lebenserhaltungs(ECLS)-Maschine oder eine Herz-Lungen-Maschine für kardiopulmonalen Bypass ist und wobei die Vorrichtung (1) zur extrakorporalen Blutbehandlung eine Vorrichtung zur kontinuierlichen Nierenersatztherapie (CRRT) für Intensivbehandlungen ist.

15. Kombination nach dem vorstehenden Anspruch 14, wobei die Maschine mit hoher Blutflussrate (36) umfasst:

einen Blutkreislauf, der eine venöse Leitung (37) und eine oxygenierte Leitung (40) umfasst, die mit Kanülen versehen sind, die dafür gestaltet sind, in Venen oder Arterien eines Patienten (P) platziert zu werden;
eine Pumpe (38);
einen Oxygenator (39);
wobei die Pumpe (38) dafür gestaltet ist, Blut durch den Blutkreislauf und durch den Oxygenator (39) mit einer Blutflussrate von mehr als 1 L/min, gegebenenfalls mehr als 2 l/min, gegebenenfalls zwischen 2 L/min und 5 L/min, zu pumpen;
wobei die Blutentnahmeleitung (6) und die Blutrückführleitung (7) der Vorrichtung (1) zur extrakorporalen Blutbehandlung mit der venösen Leitung (37) und/oder mit der oxygenierten Leitung (40) der Maschine mit hoher Blutflussrate (36) verbunden/verbindbar sind.

**Revendications**

1. Appareil de traitement sanguin extracorporelz, comprenant :

   une unité de traitement (2) ;
   un circuit sanguin extracorporel ayant une ligne de prélèvement sanguin (6) reliée à une entrée de l'unité de traitement (2) et une ligne de retour sanguin (7) reliée à une sortie de l'unité de traitement (2) ; ledit circuit sanguin extracorporel étant configuré pour être relié soit à un accès vasculaire d'un patient (P), soit à un circuit sanguin d'une machine à débit sanguin élevé (36), la machine à débit sanguin élevé (36) étant un appareil d'oxygénation par membrane extracorporelle (ECMO) ou un dispositif d'assistance extracorporelle (ECLS) ou une machine cœur-poumon pour circulation cardio-pulmonaire ; au moins un capteur de pression (25, 26) configuré pour détecter une pression dans au moins un emplacement de mesure dans le circuit sanguin extracorporel ;
   une pompe à sang (10) configurée pour réguler un débit sanguin (Qb) à travers le circuit sanguin extracorporel ;
   une unité de commande (100) reliée à la pompe à sang (10) et à l'au moins un capteur de pression (25, 26), l'unité de commande (100) étant configurée pour vérifier un type de raccordement du circuit sanguin extracorporel en réalisant la procédure suivante :

      - la réception d'au moins une pression mesurée provenant de l'au moins un capteur de pression (25, 26) ;

   **caractérisé en ce que** la procédure comporte en outre :

      - la détection du fait que le circuit sanguin extracorporel est relié à l'accès vasculaire du patient (P) ou au circuit sanguin de la machine à débit sanguin élevé (36) en réalisant une analyse de l'au moins une pression mesurée ou d'au moins un paramètre corrélé à l'au moins une pression mesurée, l'étape de détection comprenant une identification automatique du type de raccordement entre un raccordement du circuit sanguin extracorporel à l'accès vasculaire du patient et un raccordement du circuit sanguin extracorporel au circuit sanguin de la machine à débit sanguin élevé (36) ;
      - l'émission d'au moins un signal si ou lorsque le raccordement à l'accès vasculaire du patient (P) et/ou le raccordement au circuit sanguin de la machine à débit sanguin élevé (36) est détecté.

2. Appareil selon la revendication 1, dans lequel l'au moins une pression mesurée comprend une pression d'accès ($P_{acc}$, $P_{acc\_Qb0}$) et/ou une pression de retour ($P_{ret}$, $P_{ret\_Qb0}$) ;
   dans lequel l'au moins un capteur de pression (25, 26) comprend :

   un capteur de pression d'accès (25) configuré pour mesurer la pression d'accès ($P_{acc}$, $P_{acc\_Qb0}$) à un emplacement de mesure dans la ligne de prélèvement sanguin (6) ; et/ou
   un capteur de pression de retour (26) configuré pour mesurer la pression de retour ($P_{ret}$, $P_{ret\_Qb0}$) à un emplacement de mesure dans la ligne de retour sanguin (7).

3. Appareil selon la revendication 1 ou 2, dans lequel la réalisation de l'analyse comprend : la comparaison de l'au moins une pression mesurée ou de l'au moins un paramètre corrélé à l'au moins une pression mesurée avec au moins une valeur de référence.

4. Appareil selon la revendication 3, dans lequel l'au moins une pression mesurée comprend une pression statique ($P_{ret\_Qb0}$, $P_{acc\_Qb0}$) mesurée par l'au moins un capteur de pression (25, 26) lorsque le débit sanguin (Qb) à travers le circuit sanguin extracorporel est nul et dans lequel l'au moins une valeur de référence comprend une pression limite inférieure ($Lvasc_{min}$) et une pression limite supérieure ($Lvasc_{max}$) ;

dans lequel l'unité de commande (100) est configurée pour réaliser la procédure suivante :

la réception de la pression statique ($P_{ret\_Qb0}$, $P_{acc\_Qb0}$);
la réception de la pression limite inférieure ($Lvasc_{min}$) et de la pression limite supérieure ($Lvasc_{max}$) ;
la comparaison de la pression statique ($P_{ret\_Qb0}$, $P_{acc\_Qb0}$) à la pression limite inférieure ($Lvasc_{min}$) et à la pression limite supérieure ($Lvasc_{max}$) ;
dans lequel :
si la pression statique ($P_{ret\_Qb0}$, $P_{acc\_Qb0}$) est en dehors d'une plage comprise entre la pression limite inférieure ($Lvasc_{min}$) et la pression limite supérieure ($Lvasc_{max}$), alors l'unité de commande (100) enregistre que le circuit sanguin extracorporel est relié au circuit sanguin de la machine à débit sanguin élevé (36).

5. Appareil selon la revendication 3 lorsqu'elle dépend de la revendication 2, dans lequel l'au moins une pression mesurée ($P_{ret}$, $P_{ret\_Qb0}$, $P_{acc}$, $P_{acc\_Qb0}$) comprend une pression statique de retour ($P_{ret\_Qb0}$) mesurée par le capteur de pression de retour (26) et une pression statique d'accès ($P_{acc\_Qb0}$) mesurée par le capteur de pression d'accès (25), lorsque le débit sanguin ($Qb$) à travers le circuit sanguin extracorporel est nul, dans lequel l'au moins une valeur de référence comprend au moins une pression limite inférieure ($Lvasc_{min}$) et au moins une pression limite supérieure ($Lvasc_{max}$) ;
dans lequel l'unité de commande (100) est configurée pour réaliser la procédure suivante :

la réception de la pression statique de retour ($P_{ret\_Qb0}$) et de la pression statique d'accès ($P_{acc\_Qb0}$) ;
la réception de l'au moins une pression limite inférieure ($Lvasc_{min}$) et de l'au moins une pression limite supérieure ($Lvasc_{max}$) ;
la comparaison de la pression statique de retour ($P_{ret\_Qb0}$) et/ou de la pression statique d'accès ($P_{acc\_Qb0}$) avec l'au moins une pression limite inférieure ($Lvasc_{min}$) et avec l'au moins une pression limite supérieure ($Lvasc_{max}$) ;
dans lequel :
si la pression statique de retour ($P_{ret\_Qb0}$) et/ou la pression statique d'accès ($P_{acc\_Qb0}$) se trouve en dehors d'une plage comprise entre l'au moins une pression limite inférieure ($Lvasc_{min}$) et l'au moins une pression limite supérieure ($Lvasc_{max}$), alors l'unité de commande (100) enregistre que le circuit sanguin extracorporel est relié au circuit sanguin de la machine à débit sanguin élevé (36).

6. Appareil selon la revendication 5, dans lequel si la pression statique de retour ($P_{ret\_Qb0}$) et/ou la pression statique d'accès ($P_{acc\_Qb0}$) sont dans la plage comprise entre l'au moins une pression limite inférieure ($Lvasc_{min}$) et l'au moins une pression limite supérieure ($Lvasc_{max}$), alors l'unité de commande (100) vérifie si : une différence entre la pression statique d'accès ($P_{acc\_Qb0}$) et la pression statique de retour ($P_{ret\_Qb0}$) est nulle dans le cas où les hauteurs d'un emplacement de mesure de la pression d'accès effectué par le capteur de pression d'accès (25) et d'un emplacement de mesure de la pression de retour effectuée par le capteur de pression de retour (26) sont les mêmes ; ou une différence entre la pression statique d'accès ($P_{acc\_Qb0}$) et la pression statique de retour ($Pret\_Qb0$) est égale à un décalage hydrostatique (Phydr) dû à des hauteurs différentes de l'emplacement de mesure de la pression d'accès effectuée par le capteur de pression d'accès (25) et de l'emplacement de mesure de la pression de retour effectuée par le capteur de pression de retour (26) ; dans lequel :

si la différence entre la pression statique d'accès ($P_{acc\_Qb0}$) et la pression statique de retour ($P_{ret\_Qb0}$) n'est pas nulle ou si la différence entre la pression statique d'accès ($P_{acc\_Qb0}$) et la pression statique de retour ($P_{ret\_Qb0}$) n'est pas égale au décalage hydrostatique ($P_{hydr}$), alors l'unité de commande (100) enregistre que le circuit sanguin extracorporel est relié au circuit sanguin de la machine à débit sanguin élevé (36) ; ou
si la différence entre la pression statique d'accès ($P_{acc\_Qb0}$) et la pression statique de retour ($P_{ret\_Qb0}$) est nulle ou si la différence entre la pression statique d'accès ($P_{acc\_Qb0}$) et la pression statique de retour ($P_{ret\_Qb0}$) est égale au décalage hydrostatique ($P_{hydr}$), alors l'unité de commande (100) enregistre que le circuit sanguin extracorporel est relié à l'accès vasculaire du patient (P).

7. Appareil selon la revendication 3, dans lequel l'au moins un paramètre corrélé à l'au moins une pression mesurée comprend au moins un coefficient de chute de pression de cathéter ($K_{cath}$) et dans lequel l'au moins une valeur de référence comprend une valeur de référence de coefficient de chute de pression ($K_{cath\_min}$) ;
dans lequel l'unité de commande (100) est configurée pour réaliser la procédure suivante :

la réception de la valeur de référence du coefficient de chute de pression ($K_{cath\_min}$) ;
le calcul de l'au moins un coefficient de chute de pression de cathéter ($K_{cath}$) ;
dans lequel :

si l'au moins un coefficient de chute de pression de cathéter ($K_{cath}$) est inférieur à la valeur de référence du coefficient de chute de pression ($K_{cath\_min}$), alors l'unité de commande (100) enregistre que le circuit sanguin extracorporel est relié au circuit sanguin de la machine à débit sanguin élevé (36) ;

si l'au moins un coefficient de chute de pression de cathéter ($K_{cath}$) est égal ou supérieur à la valeur de référence du coefficient de chute de pression ($K_{cath\_min}$), alors l'unité de commande (100) enregistre que le circuit sanguin extracorporel est relié à l'accès vasculaire du patient (P).

8. Appareil selon la revendication 7, dans lequel l'au moins un coefficient de chute de pression de cathéter ($K_{cath}$) comprend un coefficient de chute de pression de cathéter d'accès ($K_{cath}^{acc}$) et/ou un coefficient de chute de pression de cathéter de retour ($K_{cath}^{ret}$) ;

dans lequel :

si le coefficient de chute de pression du cathéter d'accès ($K_{cath}^{acc}$) et le coefficient de chute de pression du cathéter de retour ($K_{cath}^{ret}$) sont inférieurs à la valeur de référence du coefficient de chute de pression ($K_{cath\_min}$), alors l'unité de commande (100) enregistre que le circuit sanguin extracorporel est relié au circuit sanguin de la machine à débit sanguin élevé (36) ; ou

si le coefficient de chute de pression du cathéter d'accès ($K_{cath}^{acc}$) et le coefficient de chute de pression du cathéter de retour ($K_{cath}^{ret}$) sont égaux ou supérieurs à la valeur de référence du coefficient de chute de pression ($K_{cath\_min}$), alors l'unité de commande (100) enregistre que le circuit sanguin extracorporel est relié à l'accès vasculaire du patient (P).

9. Appareil selon la revendication 8, dans lequel, si le coefficient de chute de pression du cathéter d'accès ($K_{cath}^{acc}$) est égal ou supérieur à la valeur de référence du coefficient de chute de pression ($K_{cath\_min}$) et que le coefficient de chute de pression du cathéter de retour ($K_{cath}^{ret}$) est inférieur à la valeur de référence du coefficient de chute de pression ($K_{cath\_min}$) ou inversement, alors l'unité de commande (100) enregistre un état de raccordement incertain.

10. Appareil selon la revendication 2, dans lequel la réalisation de l'analyse comprend : la vérification d'une corrélation entre la pression d'accès ($P_{acc}$) et la pression de retour ($P_{ret}$).

11. Appareil selon la revendication 10, dans lequel la vérification d'une corrélation comprend la surveillance de la pression d'accès ($P_{acc}$) et de la pression de retour ($P_{ret}$) au cours du temps pendant au moins une partie du traitement sanguin extracorporel ; dans lequel si la pression d'accès ($P_{acc}$) diminue et que la pression de retour ($P_{ret}$) augmente ou si la pression d'accès ($P_{acc}$) augmente et que la pression de retour ($P_{ret}$) diminue pendant que le débit sanguin (Qb) à travers le circuit sanguin extracorporel est maintenu constant, alors l'unité de commande (100) enregistre que le circuit sanguin extracorporel est relié au circuit sanguin de la machine à débit sanguin élevé (36).

12. Appareil selon l'une quelconque des revendications 1 à 11, dans lequel l'unité de commande (100) est configurée pour vérifier le type de raccordement au démarrage ou au redémarrage d'un traitement sanguin extracorporel et/ou au cours du traitement sanguin extracorporel.

13. Appareil selon l'une quelconque des revendications 1 à 12, dans lequel l'émission d'au moins un signal comprend :

l'émission d'un signal d'avertissement configuré pour avertir l'opérateur du type de raccordement détecté ; et/ou

la notification, à l'opérateur, de paramètres de fonctionnement selon le type de raccordement détecté ; et/ou

le réglage de l'appareil avec des paramètres de fonctionnement selon le type de raccordement détecté.

14. Combinaison d'un appareil de traitement sanguin extracorporel et d'une machine à débit sanguin élevé ; dans laquelle l'appareil de traitement sanguin extracorporel est selon l'une quelconque des revendications 1 à 13 précédentes ; dans laquelle la machine à débit sanguin élevé est un appareil d'oxygénation par membrane extracorporelle (ECMO) ou un dispositif d'assistance extracorporelle (ECLS) ou une machine cœur-poumon pour circulation cardio-pulmonaire, et dans laquelle l'appareil (1) pour le traitement sanguin extracorporel est un appareil de thérapie continue de remplacement rénal (CRRT) pour des traitements en soins intensifs.

15. Combinaison selon la revendication 14 précédente, dans laquelle la machine à débit sanguin élevé (36) comprend :

un circuit sanguin comprenant une ligne veineuse (37) et une ligne oxygénée (40) munies de canules configurées pour être placées dans des veines ou des artères d'un patient (P) ;

une pompe (38) ;

un oxygénateur (39) ;

dans laquelle la pompe (38) est configurée pour pomper du sang à travers le circuit sanguin et à travers l'oxygénateur (39) avec un débit sanguin supérieur à 1 L/min, éventuellement supérieur à 2 L/min, éventuellement entre 2 L/min et 5 L/min ;

dans laquelle la ligne de prélèvement sanguin (6) et la ligne de retour sanguin (7) de l'appareil (1) de traitement sanguin extracorporel sont reliées à, ou peuvent être reliées à, la ligne veineuse (37) et/ou à la ligne oxygénée (40) de la machine à débit sanguin élevé (36).

# FIG.1

# FIG.2

FIG.3

EP 4 353 275 B1

FIG.4

FIG.5

FIG.6

FIG.7A

FIG.7B

MEASURING STATIC ACCESS PRESSURE "$P_{acc\_Qb0}$"

MEASURING STATIC RETURN PRESSURE "$P_{ret\_Qb0}$"

RECEIVING LOWER LIMIT PRESSURE "$Lvasc_{min}$"

RECEIVING UPPER LIMIT PRESSURE "$Lvasc_{max}$"

RECEIVING LOWER ERROR "-E"

RECEIVING UPPER ERROR "+E"

$Lvasc_{min} < P_{ret\_Qb0} < Lvasc_{max}$

NO

YES

$-E < (P_{acc\_Qb0} - P_{ret\_Qb0}) < +E$

NO

YES

VASCULAR ACCESS
OF THE PATIENT

BLOOD CIRCUIT OF THE
HIGH BLOOD FLOW
RATE MACHINE

FIG.8

RECEIVING THE BLOOD FLOW RATE "Qb"

RECEIVING THE BLOOD VISCOSITY "$\mu$"

MEASURING THE RETURN AND ACCESS PRESSURES "$P_{ret\_Qb0}$, $P_{acc\_Qb0}$" WHEN THE BLOOD FLOW RATE IS ZERO

MEASURING THE RETURN AND ACCESS PRESSURES "$P_{ret}$, $P_{acc}$" WHEN THE BLOOD FLOW RATE IS STEADY AND DIFFERENT FROM ZERO

COMPUTING THE ACCESS CATHETER PRESSURE DROP COEFFICIENT "$K_{cath}^{acc}$" AND THE RETURN CATHETER PRESSURE DROP COEFFICIENT "$K_{cath}^{ret}$"

$K_{cath}^{ret} < K_{cath}^{min}$ AND $K_{cath}^{acc} < K_{cath}^{min}$

NO                                                                                    YES

VASCULAR ACCESS OF THE PATIENT

BLOOD CIRCUIT OF THE HIGH BLOOD FLOW RATE MACHINE

FIG.9

FIG.10

**EP 4 353 275 B1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2010288703 A1
- CN 113230531 A
- US 2022080093 A1
- CN 212817420 U
- US 20040084358 A1
- US 20190111199 A1
- EP 2941282 B1